# EUROPÄISCHE PATENTSCHRIFT

(11) **EP 1 024 140 B1**
(45) Veröffentlichungstag und Bekanntmachung des Hinweises auf die Patenterteilung: **31.07.2002**
(21) Anmeldenummer: 00100235.1
(22) Anmeldetag: 17.01.2000
(51) Int. Cl.: C07D 417/06

(54) **Verfahren zur N-Alkylierung von cyclischen Cyanamidinen**
Process for N-alkylation of cyclic cyanoamidines
Procédé pour la N-alkylation de cyanoamidines cycliques

(30) Priorität: 28.01.1999 DE 19904310
(43) Veröffentlichungstag der Anmeldung: 02.08.2000
(73) Patentinhaber: Bayer Aktiengesellschaft, 51368 Leverkusen (DE)
(72) Erfinder: Seifert, Hermann, Dr., 51467 Bergisch Gladbach (DE); Lantzsch, Reinhard, Dr., 42115 Wuppertal (DE); Lindner, Werner, Dr., 51067 Köln (DE); Jelich, Klaus, Dr., 42113 Wuppertal (DE)

(56) Entgegenhaltungen:
- EP-A- 0 235 725
- EP-A- 0 259 738
- GB-A- 2 126 222
- US-A- 4 616 025

## Beschreibung

Die vorliegende Erfindung betrifft ein neues Verfahren zur Herstellung von bekannten heterocyclischen Verbindungen durch Alkylierung geeigneter Vorstufen.

Derartige Alkylierungsreaktionen in aprotischen Lösungsmitteln sind bekannt (EP A2 0235 725).

Ebenfalls bekannt ist die Herstellung ungesättigter, heterocyclischer Verbindungen durch die Alkylierung von unsubstituierten Ringstickstoffatomen, die unter anderem in Alkoholen durchgeführt werden können (EP A2 0 259 738).

In diesen Fällen ist eine anschließende Reinigung des Produkts notwendig, um eine ausreichende Reinheit zu erreichen, zudem sind die mit den bekannten Verfahren zu erzielenden Ausbeuten unbefriedigend.

Es wurde nun gefunden, dass man Verbindungen der Formel (I) 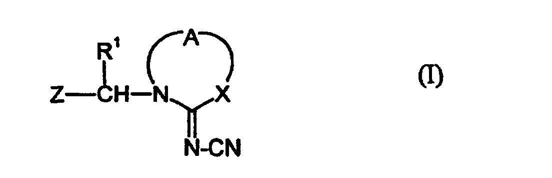 in welcher
- R¹: für ein Wasserstoffatom oder eine Alkylgruppe steht,
- A: für eine Ethylengruppe, die durch Alkyl substituiert sein kann, oder eine Trimethylengruppe, die durch Alkyl substituiert sein kann, steht,
- X: für ein Sauerstoff- oder Schwefelatom oder die Gruppe 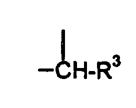 steht, in welcher
R³ für ein Wasserstoffatom oder eine Alkylgruppe steht, und
- Z: eine gegebenenfalls substituierte 5- oder 6-gliedrige heterocyclische Gruppe, die wenigstens zwei aus Sauerstoff-, Schwefel- und Stickstoffatomen ausgewählte Heteroatome enthält, oder eine gegebenenfalls substituierte 3- oder 4-Pyridylgruppe bezeichnet,
erhält, indem man Verbindungen der Formel (II) 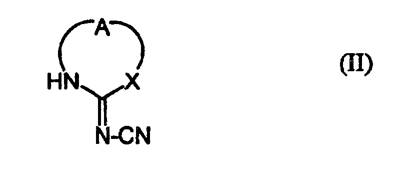 in der
A und X die oben angegebenen Bedeutungen haben,
mit Verbindungen der Formel (III) 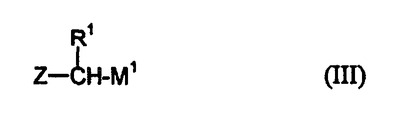 in der
- R¹ und Z: die oben angegebenen Bedeutungen haben und
- M¹: für ein Halogenatom oder die Gruppe -OSO₂-M² steht,
in der
M² eine niedere Alkylgruppe, eine Arylgruppe oder OM³ bezeichnet,
wobei
M³ eine niedere Alkylgruppe oder ein Alkalimetall bedeutet, in Gegenwart einer mit Wasser whicht oder nur teilweise mischbaren Alkohols aus der Reihe iso-Butanol, n-Butanol oder Amylalkohol und gegebenenfalls in Gegenwart einer Base umsetzt und anschließend aus einem Alkohol auskristallisiert.

Das erfindungsgemäße Verfahren vermeidet den bislang notwendigen Reinigungsschritt des Umkristallisierens und spart im Vergleich zu den Verfahren des Standes der Technik einen Arbeitsgang ein.

Die Ausbeuten, die das erfindungsgemäße Verfahren liefert, liegen überraschenderweise deutlich höher als die mit den Verfahren des Standes der Technik erzielbaren Ausbeuten.

Obwohl beim erfindungsgemäßen Verfahren die Umsetzung der Verbindungen (III) und (II) in protischen Lösungsmitteln erfolgt, wird wider Erwarten keine Etherbildung nach Williamson (s. Lehrbücher der organischen Chemie) beobachtet.

In den allgemeinen Formeln I, II und III stehen die Variablen
- R¹: bevorzugt für Wasserstoff oder eine C₁-C₃-Alkylgruppe, besonders bevorzugt für Wasserstoff;
- A: bevorzugt für eine Ethylen- oder Trimethylengruppe, die jeweils durch eine C₁-C₃-Alkylgruppe substituiert sein können, besonders bevorzugt für eine Ethylengruppe;
- x: bevorzugt für ein Sauerstoff- oder Schwefelatom, besonders bevorzugt für ein Schwefelatom,
- Z: bevorzugt für eine halogenierte, 5- oder 6-gliedrige heterocyclische Gruppe, die 2 aus der Gruppe Sauerstoff, Schwefel und Stickstoff ausgewählte Heteroatome enthält oder für eine halogenierte 3- oder 4-Pyridylgruppe, besonders bevorzugt für eine halogenierte Thiazolyl- oder 3-Pyridylgruppe, ganz besonders bevorzugt für 2-Chlor-Pyrid-5-yl;
- M: bevorzugt für Cl, Br, Tosyl oder OSO₂OK, besonders bevorzugt für Cl oder Br.

Eine ganz besonders bevorzugte Verbindung der Formel (I) ist die Verbindung der Formel (Ia) 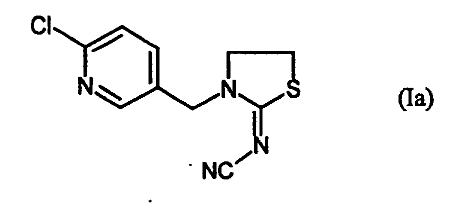 die durch Umsetzung der Verbindung der Formel 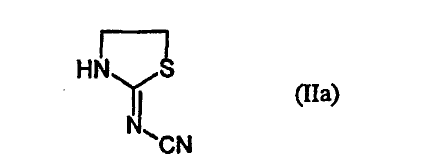 mit der Verbindung der Formel 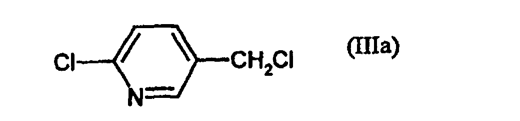 erhalten wird.

Der Begriff "niedere" steht im Zusammenhang mit Alkyl-, Alkoxy-, Alkylthio- oder Alkylsilylgruppen für C₁-C₆- bevorzugt für C₁-C₄-Alkyl-, Alkoxy-, Alkylthio- bzw. Alkylsilylgruppen.

Das erfindungsgemäße Verfahren Kann gegebenenfalls in Gegenwart von Wasser durchgeführt werden.

Als Lösungsmittel besonders bevorzugt sind mit Wasser nicht oder nur teilweise mischbare Alkohole, wie iso-Butanol oder n-Butanol oder Amylalkohol, insbesondere n-Butanol.

Das Verfahren wird gegebenenfalls in Gegenwart einer Base durchgeführt. Als Beispiele seien genannt: Alkali und Erdalkalihydroxide wie NaOH, KOH, Ca(OH)₂, Alkalimetallcarbonate oder Hydrogencarbonate wie Na₂CO₃, Li₂CO₃, K₂CO₃, Cs₂CO₃ oder NaHCO₃ und KHCO₃. Bevorzugt seien genannt K₂CO₃, NaOH und KHCO₃, insbesondere K₂CO₃.

Die Verbindungen der allgemeinen Formel II können auch als Alkali- oder Erdalkalisalz in fester oder gelöster Form eingesetzt werden.

Beim Arbeiten in Wasser oder Wasser-Alkohol-Gemischen wird das Verfahren bei einem pH-Bereich zwischen 8 und 13 durchgeführt.

Als Katalysatoren können Phasentransferkatalysatoren, gegebenenfalls quartäre Ammoniumhalogenide wie Tetrabutylammoniumchlorid etc. eingesetzt werden.

Das Verfahren kann in einem weiten Temperaturbereich durchgeführt werden, beispielsweise zwischen 30°C und 100°C, vorzugsweise zwischen 50°C und 80°C. Die Reaktion wird zweckmäßigerweise unter Atmosphärendruck durchgeführt, jedoch kann auch unter vermindertem oder erhöhtem Druck gearbeitet werden.

Bei der praktischen Durchführung des Verfahrens wird beispielsweise 1 Mol der Verbindungen der Formel (II) mit 1 bis 1,3 Mol, vorzugsweise 1 bis etwa 1,1 Mol der Verbindungen der Formel (III) bei pH 8 bis 9 in einem polaren Lösungsmittel wie Butanol in Anwesenheit von 0,6 bis 2 Mol, vorzugsweise 1 bis 1,3 Mol einer Base wie z.B. Kaliumcarbonat und gegebenenfalls in Gegenwart eines Katalysators wie Tetrabutylammoniumchlorid umgesetzt.

Die Reaktion kann auch durchgeführt werden, indem man die Verbindungen der allgemeinen Formel II als Alkali oder Erdalkalisalz gelöst oder suspendiert vorlegt und die Verbindungen der allgemeinen Formel III bei Reaktionstemperatur zudosiert.

Die Verbindungen der Formeln (II) und (III) sind aus EP 0 235 725 (und der dort zitierten Literatur) bekannt.

Die Verbindungen der Formel (I) sind beispielsweise zur Verwendung als Insektizide geeignet (EP A2 0235 752, EP A2 0259 738)

Die folgenden Beispiele erläutern den Gegenstand der Erfindung ohne sie in irgend einer Weise einzuschränken.

### Beispiel 1

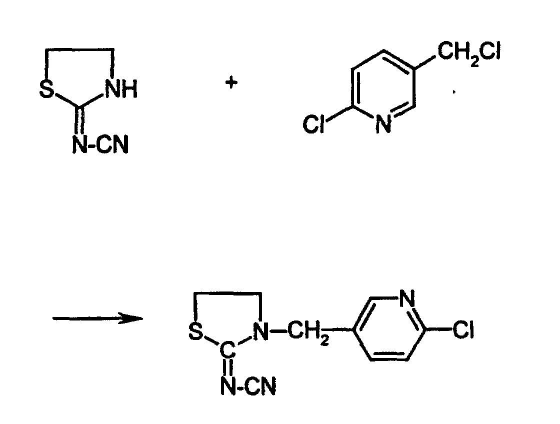

0,3 Mol 2-Cyaniminothiazolidin und 0,315 Mol 2-Chlor-5-chlormethylpyridin werden in 240 g n-Butanol gelöst und auf 80°C erhitzt. Bei dieser Temperatur werden 0,36 Mol Kaliumcarbonat zudosiert und 2 h bei 80°C gerührt. Nach Abkühlen (auf 65°C) werden 250 g Wasser zugegeben und die Phasen getrennt. Anschließend wird die organische Phase 3 h bei 50°C gerührt und anschließend innerhalb von 3 h auf 3°C abgekühlt. Ausgefallenes Produkt wird abfiltriert und getrocknet; 62,3 g (83 % der Theorie). Fp.: 135°C.

### Beispiel 2

0,3 Mol 2-Cyaniminothiazolidin und 0,315 Mol 2-Chlor-5-chlormethylpyridin und 0,015 Mol Tetrabutylammoniumbromid werden in Wasser suspendiert und auf 60°C erwärmt. Mit NaOH wird der pH-Wert des Reaktionsgemischs kontinuierlich auf 8 bis 8,5 gehalten. Nach 2 h Reaktionszeit bei 60°C wird bei dieser Temperatur eine Phasentrennung durchgeführt und die organische Phase mit 200 ml Butanol verdünnt und 3 h bei 50°C gerührt. Innerhalb von 3 h wird auf 3°C abgekühlt und ausgefallenes Produkt abgesaugt; 55,5 g (72 % der Theorie) werden so erhalten.

### Beispiel 3

39,3 g 97 %iges 2-Cyanimino-thiazolidin (=̂ 0,3 Mol) werden in 250 g 80 %igem n-Butanol (enthält 20 % H₂O) aufgenommen und mit 26,7 g 45 %iger Natronlauge (0,3 Mol) versetzt. Es entsteht eine hellgrüne Lösung, zu der bei 65°C 49,5 g 95 %iges 2-Chlor-5-chlormethylpyridin (0,29 Mol) zugegeben werden. Nach 3,5 h Reaktionszeit werden 190 g Wasser zugefügt und die Phasen getrennt. Aus der organischen Phase werden etwa 60 g n-Butanol/Wasser abdestilliert. Danach wird auf 50°C abgekühlt, Impfkristalle zugegeben und weiter auf 0°C gekühlt. Es wird 3 h bei dieser Temperatur unter Rühren kristallisiert. Durch Filtration und Trocknung werden 61,8 g 96 %iges Produkt erhalten (87 % der Theorie).

## Patentansprüche

1. Verfahren zur Herstellung von Verbindungen der Formel (I) 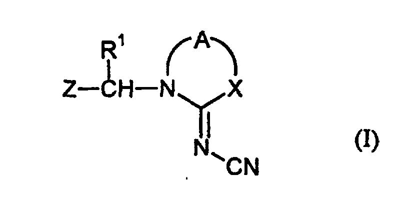 in welcher
R¹ für ein Wasserstoffatom oder eine Alkylgruppe steht,
A für eine Ethylengruppe, die durch Alkyl substituiert sein kann, oder eine Trimethylengruppe, die durch Alkyl substituiert sein kann, steht,
X für ein Sauerstoff- oder Schwefelatom oder die Gruppe 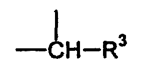 steht, in welcher
R³ für ein Wasserstoffatom oder eine Alkylgruppe steht, und
Z eine gegebenenfalls substituierte 5- oder 6-gliedrige heterocyclische Gruppe, die wenigstens zwei aus Sauerstoff-, Schwefel- und Stickstoffatomen ausgewählte Heteroatome enthält, oder eine gegebenenfalls substituierte 3- oder 4-Pyridylgruppe bezeichnet,
**gekennzeichnet dadurch, dass** man Verbindungen der Formel (II) 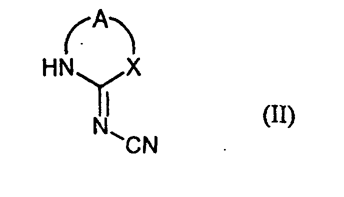 in welcher
A und X die oben angegebenen Bedeutungen haben,
mit Verbindungen der Formel (III) 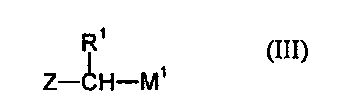 in welcher
R¹ und Z die oben angegebenen Bedeutungen haben und
M¹ für ein Halogenatom oder die Gruppe -OSO₂-M² steht,
in der
M² eine C₁-C₆-Alkylgruppe, eine Arylgruppe oder OM³ bezeichnet,
Wobei
M³ eine C₁-C₆-Alkylgruppe oder ein Alkalimetall bedeutet,
in Gegenwart eines mit Wasser nicht oder nur teilweise mischbaren Alkohols aus der Reihe iso-Butanol, n-Butanol oder Amylalkohol und gegebenenfalls in Gegenwart einer Base umsetzt.

2. Verfahren gemäß Anspruch 1, **dadurch gekennzeichnet, dass** man Verbindungen der Formel (III) einsetzt, in welcher
Z für eine halogenierte Thiazolyl- oder 3-Pyridylgruppe steht.

3. Verfahren gemäß Anspruch 1, **dadurch gekennzeichnet, dass** man Verbindungen der Formel (II), in welcher
A für eine Ethylen- oder Trimethylengruppe, die jeweils durch eine C C₃-Alkylgruppe substituiert sein können, steht,
X für ein Sauerstoff- oder Schwefelatom steht,
mit Verbindungen der Formel (III), in welcher
R¹ für Wasserstoff oder eine C₁-C₃-Alkylgruppe steht,
Z für eine halogenierte Thiazolyl- oder 3-Pyridylgruppe steht,
M¹ für Cl, Br, Tosyl oder -OSO₂OK steht,
umsetzt.

4. Verfahren zur Herstellung der Verbindung der Formel (Ia) 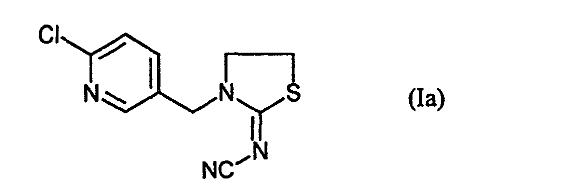 **gekennzeichnet dadurch, dass** man die Verbindung der Formel (IIa) 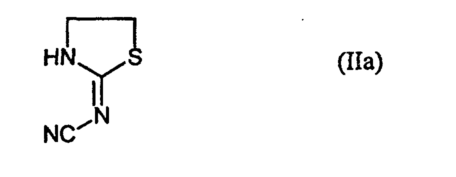 mit der Verbindung der Formel (IIIa) 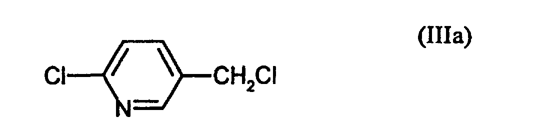 in Gegenwart eines mit Wasser nicht oder nur teilweise mischbaren Alkohols aus der Reihe iso-Butanol, n-Butanol oder Amylalkohol und gegebenenfalls in Gegenwart einer Base umsetzt.

5. Verfahren gemäß Anspruch 4, **dadurch gekennzeichnet, dass** man n-Butanol verwendet.

## Claims

1. Process for preparing compounds of the formula (I) 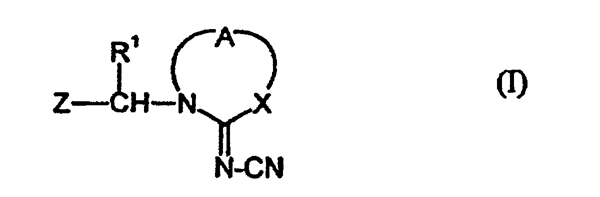 in which
R¹ represents a hydrogen atom or an alkyl group,
A represents an ethylene group, which may be substituted by alkyl, or a trimethylene group, which may be substituted by alkyl,
X represents an oxygen or sulphur atom or the group 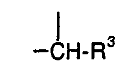 in which
R³ represents a hydrogen atom or an alkyl group, and
Z represents an optionally substituted 5- or 6-membered heterocyclic group which contains at least two heteroatoms selected from the group consisting of oxygen, sulphur and nitrogen atoms, or denotes an optionally substituted 3- or 4-pyridyl group,
**characterized in that** compounds of the formula (II) 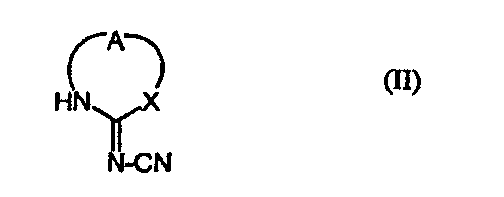 in which
A and X are each as defined above
are reacted with compounds of the formula (III) 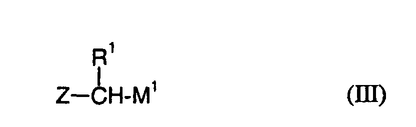 in which
R¹ and Z are each as defined above and
M¹ denotes a halogen atom or the group -OSO₂-M²,
in which
M² denotes a C₁-C₆-alkyl group, an aryl group or OM³,
where
M³ denotes a C₁-C₆-alkyl group or an alkali metal,
in the presence of an alcohol which is not or is only partly water-miscible and is selected from the group comprising iso-butanol, n-butanol and amyl alcohol, and, if appropriate, in the presence of a base.

2. Process according to Claim 1, **characterized in that** compounds of the formula (III) are used in which
Z represents a halogenated thiazolyl or 3-pyridyl group.

3. Process according to Claim 1, **characterized in that** compounds of the formula (II) in which
A represents an ethylene or trimethylene group, each of which may be substituted by a C₁-C₃-alkyl group,
X represents an oxygen or sulphur atom,
are reacted with compounds of the formula (III) in which
R¹ represents hydrogen or a C₁-C₃-alkyl group,
Z represents a halogenated thiazolyl or 3-pyridyl group,
M' represents Cl, Br, tosyl or -OSO₂OK.

4. Process for preparing the compound of the formula (Ia) 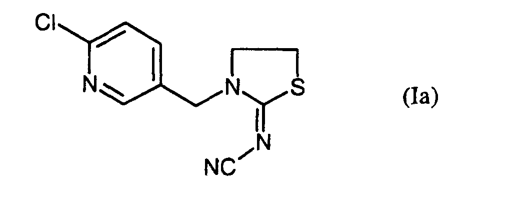 **characterized in that** the compound of the formula (IIa) 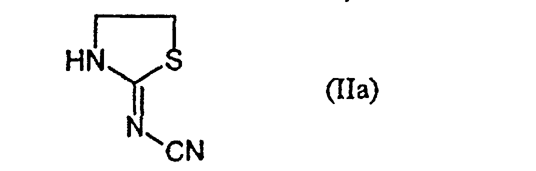 is reacted with the compound of the formula (IIIa) 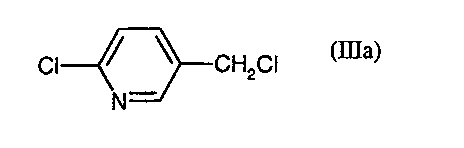 in the presence of an alcohol which is not or is only partly water-miscible and is selected from the group comprising iso-butanol, n-butanol and amyl alcohol, and, if appropriate, in the presence of a base.

5. Process according to Claim 4, **characterized in that** n-butanol is used.

## Revendications

1. Procédé de production de composés de formule (I) 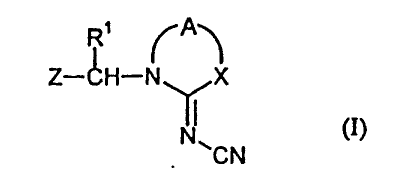 clans laquelle
R¹ représente un atome d'hydrogène ou un groupe alkyle,
A est un groupe éthylène qui peut être substitué par un radical alkyle, ou un groupe triméthylène qui peut être substitué par un radial alkyle,
X représente un atome d'oxygène ou de soufre ou le groupe 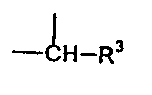 dans lequel
R³ est un atome d'hydrogène ou un groupe alkyle, et
Z est un groupe hétérocyclique pentagonal ou hexagonal éventuellement substitué, qui contient au moins deux hétéroatomes choisis entre des atomes d'oxygène, de soufre et d'azote, ou un groupe 3- ou 4-pyridyle éventuellement substitué,
**caractérisé en ce qu'**on fait réagir des composés de formule (II) 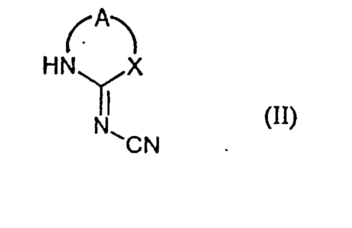 dans laquelle
A et X ont les définitions indiquées ci-dessus, avec des composés de formule (III) 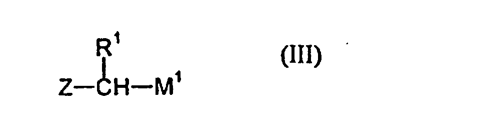 dans laquelle
R¹ et Z ont les définitions indiquées ci-dessus et
M¹ représente un atome d'halogène ou le groupe -OSO₂-M², dans lequel
M2 est un groupe alkyle en C₁ à C₆, un groupe aryle ou un groupe OM³,
dans lequel
M³ est un groupe alkyle en C₁ à C₆ ou un métal alcalin,
en présence d'un alcool non miscible à l'eau ou seulement en partie miscible à l'eau, de la série isobutanol, n-butanol ou alcool amylique et le cas échéant en présence d'une base.

2. Procédé suivant la revendication 1, **caractérisé en ce qu'**on utilise des composés de formule (III) dans laquelle
Z représente un groupe thiazolyle ou 3-pyridyle halogéné.

3. Procédé suivant la revendication 1, **caractérisé en ce qu'**on fait réagir des composés de formule (II) dans laquelle
A représente un groupe éthylène ou triméthylène dont chacun peut être substitué par un radical alkyle en C₁ à C₃,
X représente un atome d'oxygène ou de soufre,
avec des composés de formule (III) dans laquelle
R¹ est l'hydrogène ou un groupe alkyle en C₁ à C₃,
Z est un groupe thiazolyle ou 3-pyridyle halogéné,
M¹ représente Cl, Br, un groupe tosyle ou -OSO₂OK.

4. Procédé de production du composé de formule (Ia) 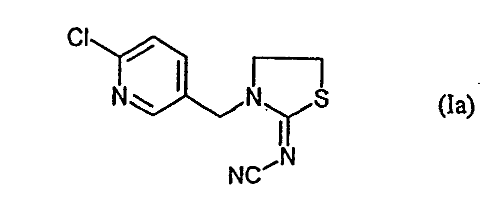 **caractérisé en ce qu'**on fait réagir le composé de formule (IIa) 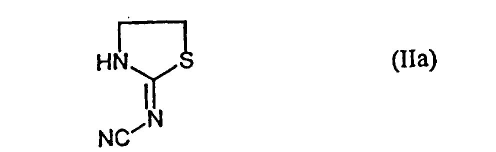 avec le composé de formule (IIIa) 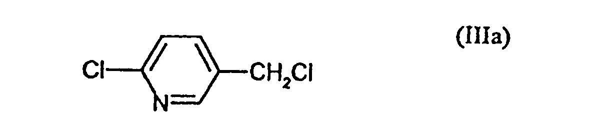 en présence d'un alcool non miscible à l'eau ou seulement en partie miscible à l'eau, de la série isobutanol, n-butanol ou alcool amylique et, le cas échéant, en présence d'une base.

5. Procédé suivant la revendication 4, **caractérisé en ce qu'**on utilise le n-butanol.
